# EUROPEAN PATENT APPLICATION

(11) **EP 2 735 559 A1**
(43) Date of publication of application: **28.05.2014**
(21) Application number: 12193763.5
(22) Date of filing: 22.11.2012
(51) Int. Cl.: C07C 51/48

(54) **Method for preparing a liquid carboxylic acid**

(71) Applicant: PURAC Biochem BV, 4206 AC Gorinchem (NL)
(72) Inventor: Van Breugel, Jan, 4206 AC Gorinchem (NL); De Haan, André Banier, 4206 AC Gorinchem (NL); Van Krieken, Jan, 4206 AC Gorinchem (NL)
(74) Representative: De Vries & Metman

(57) **Abstract**

The invention pertains to a method of preparing a carboxylic acid selected from the group consisting to propanoic acid, butanoic acid and acrylic acid, which method comprises the steps of
- providing a liquid comprising at least 5 wt.% of a dissolved inorganic salt and at least 5 wt.% of a carboxylic acid selected from the group consisting of propanoic acid, butanoic acid and acrylic acid,
- subjecting the liquid to a separation step wherein a liquid aqueous phase is separated from a liquid carboxylic acid phase.

The inorganic salt preferably is magnesium chloride.

## Description

The invention is directed to a method for preparing a liquid carboxylic acid, in particular propanoic acid, butanoic acid or acrylic acid.

Propanoic acid, butanoic acid and acrylic acid are carboxylic acids that can be manufactured via fermentation of a carbon source, such as carbohydrates or glycerol, by micro-organisms. In such a fermentation process a carbohydrate source is typically fermented by means of a micro-organism to form a carboxylic acid. The liquid wherein the carbohydrate source is fermented is called the fermentation broth or the fermentation medium.

The formation of carboxylic acid during fermentation results in a decrease of the pH of the fermentation broth. Since such a decrease in pH can be detrimental to the micro-organism's metabolic process, it is common practice to add a neutralizing agent, i.e. a base, to the fermentation medium in order to increase the pH or to maintain an optimum pH value for the micro-organism. As a result, carboxylic acid produced is typically present in the fermentation medium at least in part in the form of a carboxylate salt. Although there are micro-organisms that are to some extent resistant to acidic environments, such that fermentation can be conducted at a low pH (e.g. at a pH of 3), even in these processes at least part of the carboxylic acid is obtained as a carboxylate salt.

To recover the carboxylic acid from the fermentation broth after fermentation, downstream processing is required. In such processing, the carboxylate salt in the fermentation broth is converted into carboxylic acid, typically by addition of an acid, with simultaneous formation of an inorganic salt. The carboxylic acid and the salt then have to be separated from each other. Various processes have been described to effect this separation. For example, it is known to select the acid and the carboxylate salt in such a manner that an inorganic water-insoluble salt is formed, allowing separation of the salt from the acid. For example, acidification of a solution of a calcium carboxylate with sulphuric acid leads to the formation of water-insoluble calcium sulphate. This method has the disadvantage that the salt thus formed is often of low commercial value and leads to undesirable waste streams. Furthermore, while this process addresses removal of the salt, it still requires separation of the carboxylic acid from the aqueous medium, and as most carboxylic acids have a high solubility in water, this is not an easy process. In the art, distillation has been used, but this is an energy-consuming method.

An object of the invention is to provide a method for preparing carboxylic acid, in particular propanoic acid, butanoic acid, or acrylic acid, from an aqueous feed comprising the corresponding carboxylate salt, wherein the carboxylic acid can be isolated in an efficient manner.

A further object of the invention is to provide a method for preparing a carboxylic acid, in particular propanoic acid, butanoic acid, or acrylic acid, from an aqueous feed comprising the corresponding carboxylate salt which leads to a reduction of the formation of waste salt, a reduction of the amount of auxiliary raw materials, and/or an increase in process efficiency.

At least one of these objects is met by the method according to the invention.

The present invention pertains to a method for preparing a carboxylic acid selected from the group consisting of propanoic acid, butanoic acid and acrylic acid, which method comprises the steps of
- providing a liquid comprising at least 5 wt.% of a dissolved inorganic salt and at least 5 wt.% of a carboxylic acid selected from the group consisting of propanoic acid, butanoic acid and acrylic acid,
- subjecting the liquid to a separation step wherein a liquid aqueous phase is separated from a liquid carboxylic acid phase.

It has been found that for the specific acids propanoic acid, butanoic acid and acrylic acid the provision of a liquid comprising at least 5 wt.% of an inorganic salt and at least 5 wt.% of a carboxylic acid selected from the group consisting of propanoic acid, butanoic acid and acrylic acid, results in the formation of a two-phase liquid-liquid system, which can be subjected to a separation step wherein a liquid aqueous phase is separated from a liquid carboxylic acid phase.

In practice, the inventors surprisingly found that a suitable two-phase system could only be achieved with the method of the invention for certain carboxylic acids. Good results were obtained using a carboxylic acid selected from the group consisting of propanoic acid, butanoic acid and acrylic acid. Especially good results have been obtained with liquids containing magnesium chloride as inorganic salt. As will be discussed in more detail below, such liquids can be obtained by acidification of a magnesium carboxylate feed with hydrogen chloride. As shown in the experimental examples, a very complete phase-separation can be achieved for these reactants. It was also shown in these experiments that the dissolved inorganic salt has a distinct positive effect on phase-separation.

The process according to the invention makes it possible to isolate propanoic acid, which is also known as propionic acid, butanoic acid, also known as butyric acid, and acrylic acid, also known as propenoic acid or prop-2-enoic acid, from an aqueous medium containing these compounds without the necessity of high-energy methods like distillation, and without requiring the addition of further organic compounds, as would be the case in an extraction process. It is noted that due to their high polarity, it is difficult to find an extraction solvent which allows efficient extraction of these particular acids from an aqueous environment, and which at the same time does not bring undesirable organic contaminants into the system.

The first step in the process according to the invention is the provision of a liquid comprising at least 5 wt.% of a dissolved inorganic salt and at least 5 wt.% of a carboxylic acid selected from the group consisting of propanoic acid, butanoic acid and acrylic acid.

To obtain efficient phase separation it is preferred for the medium to contain at least 10 wt.% of inorganic salt and at least 10 wt.% of the acid to be recovered. The phase separation can be improved by increasing the salt concentration, increasing the acid concentration, or increasing both the salt concentration and the acid concentration. There is no technical maximum for the amount of acid. The maximum will be determined by the other components present in the system to arrive at 100 wt.%. For the amount of salt the maximum may be governed by viscosity considerations and by the fact that at high concentrations the solubility product for the salt may be exceeded, leading to precipitation of the salt. However, this can be addressed by appropriate technical measures. As a general maximum for the amount of acid a value of at most 80 wt.% may be mentioned, conventionally at most 60 wt.%. As a general maximum for the amount of salt a value of at most 80 wt.% may be mentioned, in particular at most 60 wt.%. The amount of water in the system will be a balance between the other compounds. It generally is at least 10 wt.%, in particular at least 20 wt.%. As a maximum, a value of at most 80 wt.% may be mentioned. It will be evident to the skilled person that the total composition of the medium adds up to 100 wt.%.

The liquid may contain further components. For example, where the acid is derived from a fermentation process, the liquid may contain biomass. For optimum processing, it is preferred for the liquid to contain at most 5 wt.% of biomass, in particular at most 3 wt.% of biomass. It is particularly preferred for the liquid to contain at most 1 wt.% of biomass.

For propionic acid it is preferred for the medium to contain at least 15 wt.% of inorganic salt and at least 20 wt.% of the acid to be recovered, more specifically at least 20 wt.% of inorganic salt and at least 25 wt.% of the acid to be recovered.

For butanoic acid it is preferred for the medium to contain at least 5 wt.% of inorganic salt and at least 10 wt.% of the acid to be recovered, specifically at least 10 wt.% of inorganic salt and at least 15 wt.% of the acid to be recovered, more specifically at least 15 wt.% of inorganic salt and at least 20 wt.% of the acid to be recovered.

For acrylic acid it is preferred for the medium to contain at least 15 wt.% of inorganic salt and at least 20 wt.% of the acid to be recovered, more specifically at least 20 wt.% of inorganic salt and at least 25 wt.% of the acid to be recovered.

The inorganic salt present in the medium comprises a cation and an anion. Preferably, the salt has a solubility in water of at least 10 g per 100 g water at 20 °C, more preferably at least 20 g per 100 g water, even more preferably at least 40 g per 100 g water at 20 °C.

In one embodiment the cation of the salt is a divalent cation, e.g., a cation of Mg, Ca, or Fe. The use of Ca or Mg is preferred, with the use of Mg being particularly preferred.

In one embodiment, the cation of the salt is a trivalent cation, e.g., Al or Fe. The use of Al is preferred.

In one embodiment the cation is a monovalent cation, e.g., Na, K, NH4, Li.

The anion is generally selected from halogenides, in particular fluoride, chloride, bromide, and iodide, more specifically a chloride, sulphate, nitrate, and phosphate. The anion preferably is chloride.

As will be discussed in more detail below, it is preferred for the salt to be magnesium chloride.

The use of salt mixtures is also envisaged. It may be preferred, however, for the salt to consist for at least 90 wt.% of MgCl2, in particular at least 95%.

The inorganic salt has to be present in the two-phase system in its dissolved state (i.e. solvated by water) to efficiently promote phase-separation. Provided that the inorganic salt has sufficient solubility, the inorganic preferably has a divalent or trivalent cation. Such cations are expected to have a more pronounced effect on phase-separation than monovalent cations. Furthermore, the size of the ions of the inorganic salt is preferably small, such as for example sodium, lithium, magnesium, fluoride and chloride. An example of an inorganic salt with both a small anion and a small divalent cation is magnesium chloride. Specific combinations of carboxylate salt and inorganic acid can be used to induce and/or enhance the formation of a two-phase system. It is important that the inorganic salt formed after acidification is soluble in water and promotes phase-separation sufficiently that a two-phase system is obtained.

The phase separation can also be improved by decreasing the reaction temperature, or by increasing the concentration of other solute salts, e.g., sodium chloride. The latter option is not preferred, however, as it will lead to the presence of additional ions in the mixture.

In the present invention, phase separation occurs. That is, two separate liquid phases are formed. The first phase in the two-phase system formed in the method of the invention is an aqueous liquid comprising dissolved inorganic salt. The second phase is a layer comprising liquid carboxylic acid. The second phase mainly consists of carboxylic acid and may further comprise some impurities. For example, the second phase typically comprises at least 60 wt.%, preferably at least 80 wt.%, more preferably at least 90 wt.%, even more preferably at least 95 wt.% of the carboxylic acid, based on the total weight of the second phase. The second phase does typically not comprise auxiliary compounds required to establish the formation of a two-phase system other than carboxylic acid. Accordingly, the second phase typically comprises essentially no organic solvent and/or amine compounds.

Of the salt present in the system, at least 90 wt.% is present in the aqueous phase, in particular at least 95 wt.%, more in particular at least 99 wt.%.

Of the acid present in the system, at least 80 wt.% is present in the aqueous phase, in particular at least 90 wt.%, more in particular at least 95 wt.%.

The term "phase-separation" as used herein refers to the formation of a two-phase system, consisting of two separate layers. For example, during acidification as will be discussed below, two liquid phases will be formed, viz. an aqueous salt solution and liquid carboxylic acid. Liquid carboxylic acid will separate from the aqueous salt solution and form a separate layer. The salt present in the aqueous salt solution is the inorganic salt.

Separating the liquid aqueous phase from the carboxylic acid phase can be done by methods known in the art for separating a liquid-liquid two-phase system. Examples of suitable apparatus and methods for liquid-liquid separation include decantation, settling, centrifugation, use of plate separators, use of coalescers, and use of hydrocyclones. Combination of different methods and apparatus may also be used.

The separation step may be carried out at any suitable temperature, in general in the range of 5-95°C. Working below ambient temperature may require cooling operations. On the other hand, a higher temperature may negatively impact the phase separation. Therefore, it may be preferred for the temperature to be at least 10°C, in particular at least 15°C, more in particular at least 20°C. Depending on the circumstances a lower limit of at least 30°C is also possible. The temperature preferably is at most 70°C, more in particular at most 50°C.

The process according to the invention yields an organic acid product stream and a liquid aqueous phase, which is an aqueous salt solution.

The organic acid product stream generally comprises at least 60 wt.%, preferably at least 80 wt.%, more preferably at least 90 wt.%, even more preferably at least 95 wt.% of the carboxylic acid, based on the total weight of the organic acid product stream. It is preferred for the water content of the organic acid product stream to be below 20 wt.%, more preferably below 10 wt.%, still more preferably below 5 wt.%. The salt content of the organic acid stream generally is at most 5 wt.%, preferably at most 1 wt.%, more preferably at most 0.1 wt.%, still more preferably at most 0.01 wt.%. If the salt content is relatively high, additional processing may be required.

The aqueous salt solution contains at least 5 wt.% of inorganic salt, generally in the solute state. More in particular, in one embodiment the solution contains at least 10 wt.% of inorganic salt, preferably at least 15 wt.%. In general, the concentration is at most 40 wt.%. The amount of acid in the aqueous salt solution is limited. In general, the amount of acid in the aqueous salt solution is below 10 wt.%, more in particular below 5 wt.%, still more in particular at most 1 wt.%.

In one embodiment, the starting liquid for the process according to the invention is obtained by providing an aqueous liquid comprising an inorganic salt of propanoic acid, butanoic acid, or acrylic acid, and acidifying the aqueous liquid using an inorganic acid to a pH at which a majority of the acid is in the acid form. Preferably, at least 90% of the acid is in the acid form, more preferably at least 95% wt.% of the acid is in the acid form. In particular at least 99 wt.% of the acid is in the acid form.

The aqueous liquid comprising an inorganic salt of propanoic acid, butanoic acid, or acrylic acid can be derived from various sources. In one embodiment, it is be derived from a fermentation process, in which case it may also be indicated as fermentation medium or fermentation broth. The broth or medium as obtained from the fermentation reactor may be subjected to intermediate processing steps, including one or more of biomass removal, concentration, and contaminant removal.

According to this embodiment of the invention, liquid carboxylic acid is prepared by the acidification of an aqueous feed comprising a carboxylate salt by an inorganic acid. The acidification of the aqueous feed by the inorganic acid will result in a mixture comprising carboxylic acid, water and an inorganic salt dissolved in the water. This mixture will phase-separate due to the immiscibility of the carboxylic acid and the aqueous inorganic salt solution. Thus, this embodiment of the invention makes it possible to use liquid-liquid separation to separate carboxylic acid from a fermentation broth. Therefore, for these specific acids it is possible to isolate the acid by acidifying an aqueous feed comprising a salt of the carboxylic acid with a mineral acid, and isolating the thus formed aqueous phase from the carboxylic acid phase.

The process according to this embodiment of the invention starts with the provision of an aqueous feed comprising a salt of a carboxylic acid selected from propanoic acid, butanoic acid and acrylic acid.

In one embodiment the salt is a carboxylate salt of a divalent metal such as Mg, Ca, and Fe. The use of Mg and Ca is preferred. The use of Mg is particularly preferred.

In one embodiment, the salt is a trivalent metal salt such as an aluminium salt or an iron(III) salt. As trivalent metal salt, the use of aluminium is preferred.

In one embodiment the salts is a monovalent salt such as a salt of Na, K, NH4, Li.

The aqueous liquid comprising the carboxylate salt may be an aqueous solution or an aqueous suspension (e.g. a slurry). The presence of solid matter in the aqueous feed is possible to a certain extent, dependent on the equipment used and the pumpability of the aqueous feed (i.e. solid matter must not prevent the aqueous feed from being pumped into the reaction vessel), as known to the skilled person. An example of solid matter that can be present in a suspension is carboxylate salt in solid form.

The concentration of carboxylate salt in the aqueous feed may be at least 50 g/l, preferably at least 100 g/l. Higher concentrations of carboxylate salt may be desirable, because these will, upon acidification, promote and/or enhance phase-separation.

For propionate it may be preferred for the aqueous liquid to contain at least 200g/l of propionate salt, preferably at least 400 g/l.

For butanoate it may be preferred for the aqueous liquid to contain at least 100 g/l of butanoate salt, preferably at least 200 g/l.

For acrylate it may be preferred for the aqueous liquid to contain at least 200 g/l of acrylate salt, preferably at least 400 g/l.

Values over 750 g/l may be undesirable, because the aqueous feed may in this case become too viscous and/or contain too much solid matter to be sufficiently pumpable. Taking this into account it is within the scope of the skilled person to determine a suitable maximum value. For an example, for magnesium propionate a preference for a maximum value of 600 g/l may be mentioned. For calcium propionate a preference for a maximum value of 400 g/l may be mentioned.

For an example, for magnesium butanoate a preference for a maximum value of 500 g/l may be mentioned. For calcium butanoate a preference for a maximum value of 250 g/l may be mentioned.

For an example, for magnesium acrylate a preference for a maximum value of 600 g/l may be mentioned. For calcium acrylate a preference for a maximum value of 400 g/l may be mentioned.

The aqueous feed may, besides carboxylate salt, also comprise certain amounts of carboxylic acid, for example because it originates from a fermentation step conducted at low pH.

The aqueous feed is acidified by contacting it with an inorganic acid. Suitable acids include hydrogen halide (*viz*. hydrogen chloride, hydrogen iodide, hydrogen bromide and hydrogen fluoride), sulphuric acid and nitric acid.

In one embodiment the acid is selected such that the cation present in the carboxylate salt and the anion of the acid form an inorganic salt which has a good solubility in water as has been described above.

Below are given a number of examples of suitable combinations of inorganic acids and carboxylate salt.

In case a hydrogen halide is used as the inorganic acid, the carboxylate salt may be a lithium, sodium, potassium, ammonium, magnesium, calcium, iron, nickel, cobalt or aluminum carboxylate. The hydrogen halide may be selected from the group consisting of hydrogen chloride (HCl), hydrogen iodide (HI), hydrogen bromide (HBr) and hydrogen fluoride (HF).

In case sulphuric acid is used as the inorganic acid, the carboxylate salt is preferably a sodium, potassium, magnesium or ammonium carboxylate.

In case nitric acid is used as the inorganic acid, the carboxylate salt may be a lithium, sodium, potassium, ammonium, magnesium, calcium or iron carboxylate.

In this embodiment, the inorganic salt in the liquid is the salt formed in the acidification reaction, i.e. the reaction between the inorganic acid and the carboxylate salt. Accordingly, the cation of the inorganic salt will be the same as that of the carboxylate salt and the anion of the inorganic salt will be the same as that of the inorganic acid.

In one embodiment of the present invention, the inorganic salt is thermally degradable and the inorganic salt solution is subjected to a temperature of at least 300 °C, thereby decomposing the inorganic salt. Such a heat treatment step is herein referred to as thermal decomposition, thermal hydrolysis, thermohydrolysis or thermal salt splitting, which terms all refer to the same process and may be used interchangeably. Thermal decomposition of the inorganic salt may be desirable for decreasing the waste production in the process by converting the inorganic salts to valuable products. The use of thermal decomposition may even result in a net process that produces essentially no salt waste. The phase-separation achieved by the method of the invention is especially suitable when one wants to conduct a subsequent thermal decomposition step, because the separated aqueous phase comprising the inorganic salt has a high purity. Accordingly, it can be directly fed to a thermohydrolysis unit without the need for additional treatment of the aqueous phase. Nevertheless, it may still be desirable to subject the aqueous phase to one or more further purification steps to remove any solids or other impurities prior to thermal decomposition and/or to recover residual amounts of organic acid. Other processing steps such as concentration or dilution may also be envisaged.

The efficiency of the thermal decomposition step *i.a.* depends on the purity of the separated aqueous phase. Therefore, it is desirable that the phase-separation is as complete as possible, such that the aqueous phase does comprise no or only very small amounts of carboxylic acid. In case carboxylate or carboxylic acid would be present in the separated aqueous phase, this would result in a decrease in the final recovery yield, because the carboxylate or carboxylic acid will be incinerated under the high temperatures used in this step.

Examples of thermally degradable inorganic salts are magnesium chloride, iron chloride, nickel chloride, cobalt chloride and iron nitrate. For example, magnesium chloride may be obtained in the aqueous phase by acidifying a magnesium carboxylate with hydrogen chloride. Iron nitrate may be obtained by acidifying an iron carboxylate with nitric acid.

In case a subsequent thermal decomposition step is conducted on the aqueous phase, the separated aqueous phase subjected to thermohydrolysis preferably has an inorganic salt concentration of 20-40 wt.%, based on the total weight of the separated aqueous phase. Too high amounts of inorganic salt present in the separated aqueous phase may result in precipitation of inorganic salt upon entering the thermohydrolysis unit. However, too low amounts of magnesium chloride present in the separated aqueous phase will result in very high energy costs. A suitable inorganic salt concentration may for example be 25-35 wt.%, which range is in particular preferred when the inorganic salt is magnesium chloride.

Thermohydrolysis is preferably conducted in the presence of inert gases, for example in the presence of dry air and/or nitrogen, such that the gas feed obtained from thermohydrolysis comprises a significant amount of such gases. Suitable apparatuses for conducting thermal decomposition are known in the art. For example, a spray roaster or a fluid bed roaster can be used. Such apparatuses can for example be obtained at SMS Siemag.

Good results have been obtained with the thermal decomposition of magnesium chloride. Accordingly, the method of the invention may comprise the step of thermally decomposing a separated aqueous phase comprising magnesium chloride at a temperature of at least 300 °C, thereby forming magnesium oxide and hydrogen chloride. In this case, the aqueous phase to be decomposed preferably has an inorganic salt concentration of 25-35 wt.%.

The hydrogen chloride formed in the thermal decomposition of a magnesium chloride solution will be in gaseous form, typically as part of a gas comprising gaseous hydrogen chloride and gaseous water. In one embodiment, the gaseous hydrogen chloride is used to manufacture a HCl solution, which can, e.g., be used in the acidification step as described above. In another embodiment, the hydrogen chloride is used in gaseous form to acidify the carboxylate in the aqueous feed. Where HCl is applied in gaseous form, it may be desirable to conduct the acidification at temperatures higher than 80 °C. Accordingly, in this embodiment the acidification is preferably conducted at a temperature of at least 50°C, more preferably at least 80°C, for example a temperature of 80-120°C. The temperature at which acidification is conducted is mainly determined by the temperature of the aqueous feed, in particular when a gas feed is used to acidify the aqueous feed. Accordingly, the above ranges for the temperature also apply for the temperature of the aqueous feed. Thus, in this embodiment the aqueous feed may have a temperature of at least 20°C, preferably at least 50°C, more preferably at least 80°C, for example a temperature of 80-120°C.

The metal oxide formed during thermal decomposition may be used in a fermentation process, for example as a neutralizing agent or as a precursor thereof. At least part of the metal oxide may for this purpose be brought into contact with water to obtain a slurry comprising metal hydroxide (e.g. Mg(OH)₂) and/or metal oxide (e.g. MgO). By varying the composition of the MgO and/or Mg(OH)₂ in the slurry, the fermentation process can be optimized. The preferred composition of the mixture depends on the fermentation conditions and the type of microorganism used in the fermentation. Magnesium oxide may also be directly added to the fermentation broth of a fermentation process.

The type of reaction vessel wherein the acidification is conducted is not critical and the skilled person will be able to choose a suitable vessel. The vessel preferably allows for decanting the two phases (although the separation step can also be conducted in a separate vessel). In case a gas is used for acidification, the reaction vessel should be suitable for absorption of the acidic gas (e.g. hydrogen halide) in an aqueous liquid. Due to the acidic conditions of the acidification step, the reaction vessel is preferably made from acid-resistant material such as plastic or suitable duplex steel grades. The shape of the reaction vessel is not essential. The acidic gas is typically fed to the unit at or near its bottom, while the aqueous feed is fed to the column at or near its top. Examples of suitable reaction vessels are columns (e.g. packed columns, bubble columns), scrubbers (*e.g*. venture scrubbers), tray absorbers and stirred tanks. Acidification is typically conducted by bringing the aqueous feed into contact with the inorganic acid. The inorganic acid may be in the form of a gas (*e.g*. gaseous HCl) or in the form of an aqueous solution (e.g. hydrochloric acid).

If an aqueous inorganic acid solution is used in the acidification step, it preferably comprises at least 5 wt.%, more preferably at least 10 wt.% and even more preferably at least 20 wt.% inorganic acid. High concentrations are desirable, because it will result in high concentrations of inorganic salt, which promote the formation of the two-phase system.

In a preferred embodiment, gaseous hydrogen halide is used to acidify the aqueous feed. In this case, the aqueous feed is brought into contact with a gas feed comprising gaseous hydrogen halide. The hydrogen halide is then absorbed in the aqueous feed. Good results were obtained using hydrogen chloride as the hydrogen halide.

The inventors found that by conducting the acidification using gaseous hydrogen halide, the amount of hydrogen halide that can be absorbed by the aqueous feed is increased by the reaction of hydrogen halide with the carboxylate salt. When acidifying with an aqueous hydrogen halide solution, the amount of hydrogen halide that can be absorbed is limited by the azeotrope of the hydrogen halide and water. For example, the azeotrope of hydrogen chloride and water comprises 21.8 wt.% HCl at 81 °C, 20.2 wt.% HCl at 109 °C and 19.7 wt.% HCl at 116 °C. By using gaseous hydrogen halide in the method of the invention, the water/hydrogen halide azeotrope is effectively broken without expensive equipment or expending significant energy. By bringing the aqueous feed into contact with the gas feed, gaseous hydrogen halide will be absorbed by the aqueous feed without adding significant amounts of water. Thus, the aqueous feed will not be significantly diluted during acidification. In particular, the amount of water added during acidification is reduced compared to using an aqueous hydrogen halide solution. This will result in a higher concentration of halide salt in the first layer, which promotes the formation of the two-phase system. Furthermore, since a less diluted aqueous phase is obtained, a smaller amount of water will need to be evaporated during downstream processing of this phase.

The gas feed may comprise at least 1 wt.%, preferably at least 2 wt.%, more preferably at least 5 wt.% hydrogen halide, based on the total weight of the gas. A concentration of less than 1 wt.% is generally undesirable, because such a concentration requires the use of very large gas pipes to feed the gas feed to the aqueous feed in order to maintain an efficient acidification. Although high concentrations of hydrogen halide in the gas feed are generally desirable, the gas feed will in practice comprise 20 wt.% or less hydrogen halide. A suitable concentration of hydrogen halide in the gas feed is 7-12 wt.%. The hydrogen halide concentration of a gas obtained in thermohydrolysis of e.g. magnesium chloride typically falls within this range.

The temperature of the gas feed is not particularly critical. It is preferably 20 °C or higher, more preferably higher than 75 °C. At temperatures lower than 20 °C, almost all water present in the gas feed will condense, which is generally undesirable. At temperatures of 75 °C or lower, still too much water condensation may occur, depending on the concentration requirements of the separation process of the inorganic salt and the carboxylic acid. Furthermore, the gas feed preferably has a temperature of 150 °C or less. Higher temperatures will require expensive equipment for conducting the absorption, e.g. made from highly acid/corrosion resistant and temperature resistant construction material. The gas feed may for example have a temperature of 80-120 °C. The temperature of the gas feed is preferably lower than the temperature of the carboxylate feed. Preferably, the temperature of the gas feed is 1-50 °C, more preferably 3-25 °C, for example 5-15 °C lower than the temperature of the carboxylate feed. Such a temperature difference may enhance the prevention of condensation of gaseous water in the region where the gas feed enters the reaction vessel.

The use of gaseous hydrogen halide is particularly suitable for efficiently processing streams comprising both gaseous hydrogen halide and gaseous water. Accordingly, when a gas feed is used in the acidification step, the gas feed may further comprise gaseous water. The inventors realized that hydrogen halide obtained from industrial processes often comprise significant amounts of gaseous water. For example, the HCl gas stream obtained in the thermohydrolysis of magnesium chloride typically comprises an amount of water of about 25-50 wt.% based on total weight. The gas feed may have a hydrogen halide to water weight ratio (HH/H₂O ratio) that is between 1:10 and 10:1, e.g. between 1:6 and 1:3. However, in practice, the upper limit is usually determined by the hydrogen halide / water azeotrope. In case of hydrogen chloride, the upper limit of the HCl/H₂O weight/weight ratio may for example be at most 1:4.

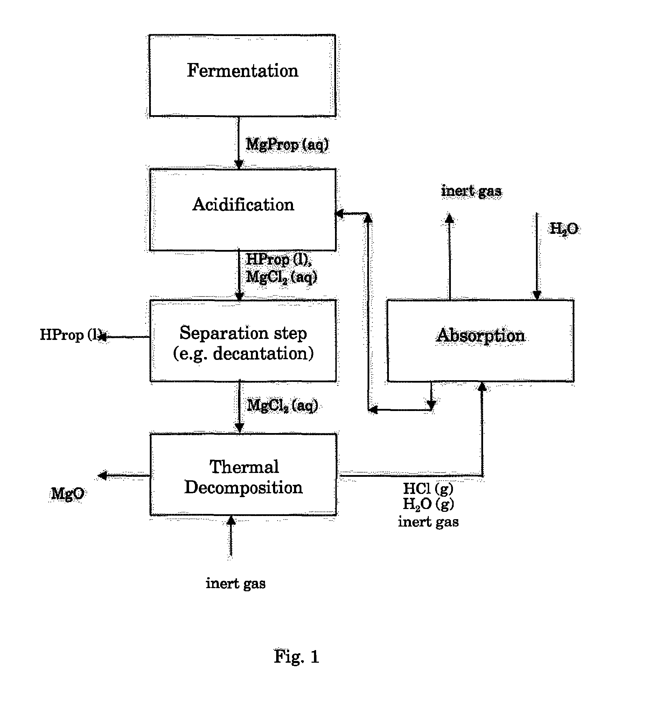

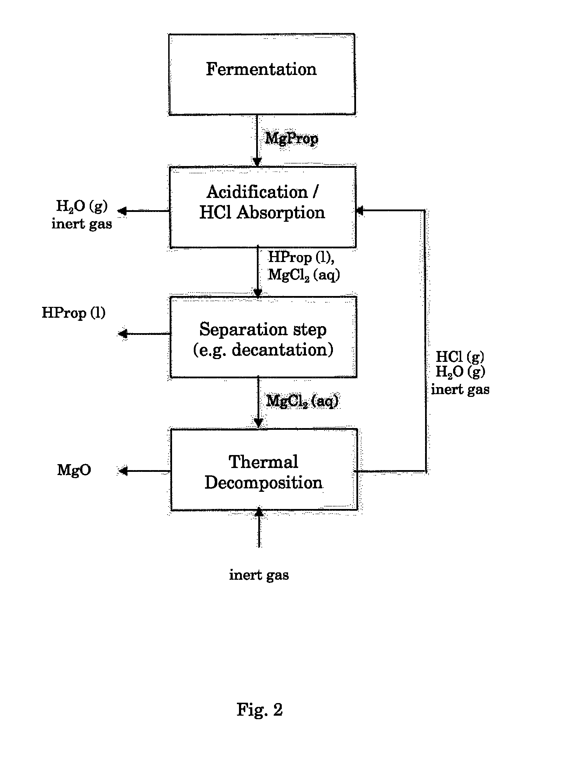

The method of the invention is preferably a continuous process, as for example illustrated by the embodiments depicted in Fig. 1 and Fig. 2. Nevertheless, it may be conducted as a batch process.

The method may further comprise a fermentation step, wherein an aqueous feed comprising a carboxylate salt is formed. Such a step typically comprises the substeps of fermenting a carbon source by means of a micro-organism to form a fermentation medium comprising a carboxylic acid, e.g., propanoic acid and (partially) neutralizing the fermentation medium in order to establish a desirable pH by adding a neutralizing agent, preferably a magnesium base, to form the carboxylate salt, e.g., propanoate. Subsequently, biomass may be separated from the fermentation medium, for example by (ultra)filtration, centrifugation or decantation of the biomass or by precipitation of the carboxylate salt (typically magnesium carboxylate) from the fermentation medium. The neutralizing agent is preferably a magnesium base. As described above, magnesium oxide or iron oxide obtained in the thermal decomposition step can be recycled in the precipitation step as a neutralizing agent or precursor thereof.

The present invention also pertains to an integrated process for the manufacture of carboxylic acid comprising the steps of
- subjecting a feed to fermentation under fermentation conditions in the presence of a microorganism to provide an aqueous medium comprising a magnesium salt of propionic acid, butanoic acid, or acrylic acid;
- subjecting the aqueous medium to an acidification step using hydrogen chloride to provide a liquid comprising at least 5 wt.% of a dissolved inorganic salt and at least 5 wt.% of a carboxylic acid selected from the group consisting of propanoic acid, butanoic acid and acrylic acid,
- subjecting the liquid to a separation step wherein a liquid aqueous phase is separated from a liquid carboxylic acid phase,
- subjecting the liquid aqueous phase, which comprises magnesium chloride in water to a thermal decomposition step at a temperature of at least 300°C to form MgO and HCl, and
- recycling HCl to the acidification step, either directly, or with an intermediate adsorption step.

For the individual steps, reference is made to what is disclosed above.

Figures 1 and 2 illustrate two embodiments of the integrated process. It should be noted that these embodiments should not be considered limiting to the invention in any way. Figure 1 shows and embodiment according to the invention, wherein a hydrochloric acid solution is used to acidify an aqueous feed comprising magnesium propanoate and form a two-phase system. The resulting aqueous phase comprising magnesium chloride is subjected to thermohydrolysis, thereby producing the gas feed which can be used in the acidification step. Figure 2 shows a similar embodiment, wherein a gas feed comprising gaseous hydrochloric acid is used instead of a hydrogen chloride solution.

The invention will further be illustrated in the following experimental examples, without being limited thereto or thereby.

### Example 1: Phase-separation of carboxylic acid in a salt solution

In this example, the possibility of isolating carboxylic acid through phase separation from solutions containing an inorganic salt and an organic acid was investigated for various acids. Each of the carboxylic acids tested are well miscible with water at 25°C under atmospheric pressure.

A 30 wt% MgCl₂ solution (expressed as anhydrate) in water was prepared by dissolution of 128.2 gram magnesium chloride hexahydrate crystals in 72.4 gram water. For a number of carboxylic acids, 10 grams of this solution were combined with an equivalent amount (= 2 mol/mol Mg²⁺) of carboxylic acid in a 20 ml headspace vial. The samples were stirred magnetically for at least 30 minutes at room temperature and allowed to settle. An overview of the samples thus created is given in Table 1. Table 1 also shows whether or not phase separation occurred

**Table 1: Overall composition of liquids with various acids and MgCl₂ as inorganic salt (in wt.%).**

| | [acid] (wt.%) | [MgCl₂] (wt.%) | phase separation? |
|---|---|---|---|
| Formic acid | 22.4 | 23.3 | no |
| Acetic acid | 27.3 | 21.8 | no |
| Propanoic acid | 31.8 | 20.5 | yes |
| Butanoic acid | 35.6 | 19.3 | yes |
| Acrylic acid | 31.2 | 20.6 | yes |
| Lactic acid | 36.2 | 19.1 | no |
| Glycolic acid (hydroxy acetic acid) | 32.6 | 20.2 | no |
| 2-hydroxybutanoic acid | 39.5 | 18.1 | no |
| Pyruvic acid | 35.7 | 19.3 | no |
| Levulinic acid (4-oxopentanoic acid) | 42.2 | 17.3 | no |
| 2-chloroacetic acid | 37.2 | 18.8 | precipitate |

Only three of the carboxylic acid samples resulted in a liquid/liquid two-phase-separation, namely propanoic acid, butanoic acid and acrylic acid. In the 2-chloroacetic acid sample, precipitation occurred, while the carboxylic acids in the other samples remained miscible with the magnesium chloride solution.

### Example 2

Additional samples were prepared for propanoic acid, butanoic acid, acrylic acid and 2-chloroacetic acid using a 20 wt% MgCl₂ solution. Samples were prepared by mixing a 20 wt% MgCl₂ solution (expressed as anhydrate) in water (prepared by dissolution of 42.7 gram hexahydrate crystals in 57.3 gram water) 12 grams of this solution were combined with an equivalent amount (= 2 mol/mol Mg²⁺ ) of carboxylic acid in a 20 ml headspace vial. The samples were stirred magnetically for at least 30 minutes at room temperature and allowed to settle. The four samples are summarized in Table 2.

**Table 2: Overall composition of liquids with various acids and MgCl₂ as inorganic salt (in wt.%).**

| | [acid] (wt.%) | [MgCl₂] (wt.%) | phase separation? |
|---|---|---|---|
| Propanoic acid | 23.7 | 15.3 | yes |
| Butanoic acid | 26.9 | 14.6 | yes |
| Acrylic acid | 23.2 | 15.4 | yes |
| 2-chloroacetic acid | 28.4 | 14.3 | no |

Again, liquid/liquid phase-separation occurred for propanoic acid, butanoic acid and acrylic acid. The sample with 2-chloroacetic acid remained a single phase; no precipitation occurred.

### Example 3: Analysis of the Two-Phase System

All samples forming a liquid/liquid two-phase system obtained in Examples 1 and 2 were analysed as follows. The acid layer was analysed on water content and Mg-content, the remainder being the carboxylic acid content. The aqueous MgCl₂-phase was analysed on carboxylic acid content. The results are shown in Tables 3 and 4.

**Table 3: Analytical results of the acid phase and the water phase of the samples of Example 1 for propanoic acid, butanoic acid and acrylic acid**

| | [acid] (wt.%) | [MgCl2] (wt.%) | H2O (wt. %) |
|---|---|---|---|
| propanoic acid - water phase | 4.2 | | |
| propanoic acid - acid phase | 96.1 | 0.007 | 3.9 |
| butanoic acid - water phase | 1.5 | | |
| butanoic acid - acid phase | 98.1 | 0.017 | 1.9 |
| acrylic acid - water phase | 5.5 | | |
| acrylic acid - acid phase | 94.3 | 0.029 | 5.7 |

**Table 4: analytical results of the acid phase and the water phase of the samples of Example 2 for propanoic acid, butanoic acid and acrylic acid**

| | [acid] (wt.%) | [MgCl2] (wt.%) | H2O (wt. %) |
|---|---|---|---|
| water phase | 9.9 | | |
| propanoic acid - acid phase | 84.6 | 0.278 | 15.1 |
| butanoic acid - water phase | 2.6 | | |
| butanoic acid - acid phase | 93.7 | 0.040 | 6.3 |
| acrylic acid - water phase | 13.0 | | |
| acrylic acid - acid phase | 78.9 | 0.948 | 20.2 |

The difference between the compositions of Example 1 and Example 2 is that the compositions of Example 1 have a higher salt concentration than those of Example 2. From a comparison between Tables 3 and 4 it can be seen that the liquids with a higher salt concentration show less acid remaining in the water phase, and a lower salt concentration and water concentration in the acid phase. This illustrates that an increase in salt concentration leads to an improved separation.

### Example 4: Phase-separation at different concentrations

A 37% HCl solution was added at room temperature to an aqueous solution comprising 50 wt.% magnesium propionate (MgProp) in a sufficient amount to effect neutralisation. The experiment was repeated using a more concentrated magnesium propionate solution (70 wt.%). In both experiments, phase-separation occurred and a two-phase system was formed.

The overall composition of the various liquids formed was as follows:

| Ex | starting liquids | | product liquid | | |
|---|---|---|---|---|---|
| | [MgProp] (wt.%) | [HCl] (wt.%) | [prop acid] (wt.%) | [MgCl2] (wt.%) | phase separation? |
| 1 | 50 | 37 | 30 | 18 | yes |
| 2 | 70 | 20 | 31 | 19 | yes |

## Claims

1. A method for preparing a carboxylic acid selected from the group consisting of propanoic acid, butanoic acid and acrylic acid, which method comprises the steps of
- providing a liquid comprising at least 5 wt.% of a dissolved inorganic salt and at least 5 wt.% of a carboxylic acid selected from the group consisting of propanoic acid, butanoic acid and acrylic acid,
- subjecting the liquid to a separation step wherein a liquid aqueous phase is separated from a liquid carboxylic acid phase.

2. Method according to claim 2, wherein the liquid comprises at least 10 wt.% of inorganic salt and at least 10 wt.% of organic acid.

3. Method according to claim 2, wherein the acid is propanoic acid, with the medium containing at least 15 wt.% of inorganic salt and at least 20 wt.% of acid, more specifically at least 20 wt.% of inorganic salt and at least 25 wt.% of acid.

4. Method according to claim 1, wherein the acid is butanoic acid, with the medium containing at least 5 wt.% of inorganic salt and at least 10 wt.% of acid, specifically at least 10 wt.% of inorganic salt and at least 15 wt.% of acid, more specifically at least 15 wt.% of inorganic salt and at least 20 wt.% of acid.

5. Method according to claim 2, wherein the acid is acrylic acid, with the medium containing at least 15 wt.% of inorganic salt and at least 20 wt.% of acid, more specifically at least 20 wt.% of inorganic salt and at least 25 wt.% of acid.

6. Method according to any one of the preceding claims, wherein the salt is a salt of magnesium, calcium, lithium, sodium, potassium and ammonium, in particular a magnesium salt.

7. Method according to any one of the preceding claims, wherein the salt is a halogenide, in particular a fluoride, chloride, bromide, or iodide, or a sulphate or nitrate salt, in particular a chloride salt.

8. Method according to any of the preceding claims, wherein the inorganic salt is a thermally degradable salt and the method further comprises the step of thermally decomposing the aqueous phase at a temperature of at least 300 °C.

9. Method according to claim 9, wherein the thermally degradable salt is magnesium chloride, which is decomposed into magnesium oxide and hydrogen chloride, and wherein the magnesium oxide is used as base or precursor of a base in a fermentation reaction to produce the carboxylic acid.

10. Method according to any one of the preceding claims, which comprises an acidification step comprising adding an inorganic acid to a solution of a salt of a carboxylic acid selected from the group consisting of propanoic acid, butanoic acid and acrylic acid to provide a liquid comprising a dissolved inorganic salt and carboxylic acid.

11. Method according to claim 10, wherein the inorganic acid provided to the acidification step is hydrogen chloride, which is derived from the thermal decomposition of magnesium chloride.

12. Method according to claim 10 or 11, wherein the acidification step is conducted at a temperature of at least 20°C, preferably at least 50°C, more preferably at least 80°C.

13. Method according to any one of the preceding claims, wherein the phase separation yields an organic acid product stream which comprises at least 60 wt.%, preferably at least 80 wt.%, more preferably at least 90 wt.%, even more preferably at least 95 wt.% of the carboxylic acid, which stream has a water content of below 20 wt.%, more preferably below 10 wt.%, still more preferably below 5 wt.%, and a salt content of at most 5 wt.%, preferably at most 1 wt.%, more preferably at most 0.1 wt.%, still more preferably at most 0.01 wt.%.

14. Method according to any one of the preceding claims, wherein the amount of acid in the liquid aqueous phase is below 10 wt.%, more in particular below 5 wt.%, still more in particular at most 1 wt.%.

15. Method according to any of the previous claims, wherein the method further comprises the step of preparing an aqueous feed comprising a salt of a carboxylic acid selected from propanoic, butanoic, and acrylic acid through a fermentation step.
